(19) Europäisches Patentamt
European Patent Office
Office européen des brevets

(11) Publication number: **0 105 481**
**A1**

(12) **EUROPEAN PATENT APPLICATION**

(21) Application number: 83109769.6

(22) Date of filing: 29.09.83

(51) Int. Cl.³: C 07 C 103/52
C 12 N 15/00, C 12 P 21/00
A 61 K 39/135, C 12 P 19/30

(30) Priority: 30.09.82 GB 8227860
03.12.82 GB 8234638

(43) Date of publication of application:
18.04.84 Bulletin 84/16

(84) Designated Contracting States:
BE CH DE FR GB LI NL SE

(71) Applicant: THE WELLCOME FOUNDATION LIMITED
183-193 Euston Road
London NW1 2BP(GB)

(72) Inventor: Boothroyd, John Charles
23 Cleveland Road Penge
London, S.E. 20(GB)

(72) Inventor: Makoff, Andrew Joseph
62 Melrose Avenue Norbury
London, S.W.16(GB)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

(54) **Novel antigens and vaccines containing them.**

(57) Novel antigenic peptides effective in stimulating immunity to foot and mouth disease virus (FMDV) are disclosed, together with methods for their preparation, vaccines containing the peptides and to their use in the prophylaxis of FMDV.

EP 0 105 481 A1

## DESCRIPTION

## NOVEL ANTIGENS AND VACCINES CONTAINING THEM

The present invention relates to novel antigenic polypeptides effective in stimulating immunity to foot and mouth disease virus (FMDV), to methods for the preparation thereof, to vaccines containing the antigen and to the use of the antigen and vaccines in the prophylaxis of FMD.

Foot and mouth disease (FMD) is one of the most virulent and contagious diseases of farm animals. The disease is endemic in several areas of the world and can be found in many countries of Africa, Asia and South America where it is controlled to varying degrees by immunisation programmes. Countries which are free of the disease remain so only by strict import and quarantine controls, together with the use of slaughter when outbreaks occur.

FMDV is an RNA (ribonucleic acid) virus classified as a member of the genus Apthovirus of the family Picornaviridae (see Cooper, P.D., et al, Intervirology, 10, 165-180, 1978). There are seven known serotypes of FMDV, the European serotypes A,O and C, the South Africa Territories serotypes SAT 1, SAT 2, and SAT 3, and the Asia 1 serotype. A number of antigenically distinct subtypes are recognised within each of these serotypes and, as the subtypes are so distinct, immunologically specific subtype vaccines are required. For each serotype or subtype several genetically distinct variants exist.

Vaccines currently used for the prophylaxis of FMD employ inactivated whole virus particles.

Foot and mouth disease virus comprises a single strand of RNA and four polypeptides, namely $VP_1$, $VP_2$, $VP_3$ and $VP_4$, which form the capsid proteins of the virus. The protein referred to here as $VP_1$ is variously referred to by other workers in the field as $VP_3$, $VP_{Thr}$, and $VP_T$ ( see Bachrach, H.L. et al, J.Immunology, 115, 1636-1641, 1975; Strohmaier, K. et al, Biochem. Biophys. Res. Comm., 85, 1640-1645, 1978; Bachrach, H.L. et al, Intervirology, 12, 65-72, 1979 ). Each of the polypeptides $VP_1$, $VP_2$, and $VP_3$ has a molecular weight of about 26,000, whilst $VP_4$ has a molecular weight of about 8,000 and it is generally considered that there are approximately 60 copies of each of them in the virus. The other polypeptides translated from the virus RNA probably have a role in virus replication.

The single strand of FMDV RNA has a molecular weight of about $2.6 \times 10^6$ which is equivalent to about 8000 nucleotides, and is of positive polarity acting as a template for both translation into polypeptides and RNA synthesis. One of the primary translational products is a protein designated P88 which is subsequently cleaved to produce the four capsid proteins $VP_1$ to $VP_4$.

Capsid protein $VP_1$, mentioned above, is susceptible to cleavage when intact virus is treated with trypsin, resulting in a large decrease in infectivity of most strains of FMDV ( Wild, T.F. and Brown, F., J. Gen. Virology, 1, 247-250, 1967). Trypsin treatment may also reduce the capacity of virus to stimulate the production of neutralising antibody. Thus it appears that $VP_1$ is likely to be the primary immunogen capable of eliciting effective protection against infection by FMDV and indeed $VP_1$ separated from virus particles produces neutralising antibody and elicits effective protection against the virus ( Laporte, J. et al, C. R. Acad. Sc. Paris, t. 276 Serie D, 3399-3401, 1973; Bachrach, H.L. et al, J. Immunology, 115, 1636-1641, 1975. ). Separation of the naturally occurring FMDV capsid proteins, particularly $VP_1$, in order to provide a safer vaccine has necessitated the use of strongly denaturing conditions and has generally been held by those skilled in the art to be disadvantageous for the maintenance of optimum immunogenicity and production of an effective vaccine.

More recently it has been shown that certain regions within $VP_1$ have been shown to be of major significance in providing the antigenic variation of the sub-types of FMDV; It has further been shown that short peptides corresponding to certain of these regions are, in their own right, effective as immunogens against FMDV (see for example J.L.Bittle et. al, Nature, 1982, 298, 30-33).

There have now been identified a number of novel peptides which have useful immunogenic properties or confer such properties upon larger peptides or polypeptides when incorporated therein.

The invention therefor provides, in a first aspect, a novel peptide characterised in that it comprises the amino acid sequence of an antigenic site of a VP1 protein selected from the serotypes $A_{24}$ Cruzeiro, $C_3$ Indaial or $O_1$BFS.

Included within the scope of the invention are fragments of the peptides containing at least 6 amino acids and derivatives of said peptides or fragments.

In particular the invention is concerned with novel peptides corresponding to the following regions, the numbering corresponding to that shown in figure 4, :- 42-61, 79-85, 95-102, 129-160, (or 162) 170-176 and 193-204 (01209) and fragments and derivatives thereof.

Of these peptides those corresponding to regions 79-85, 129-160 (or 162) and 193-204 are preferred.

Within the peptides corresponding to regions 42-61 and 129-160 (or 162) fragments and derivatives thereof which correspond to regions 42-51 and 56-61 and 129-147, 151-160 and 143-162 respectively are of particular interest.

Novel peptides within the scope of the invention include for example:-

| | |
|---|---|
| (a) VVRHEGN | (hereinafter referred to as Peptide 1) |
| (b) AVTHTGK | (hereinafter referred to as Peptide 2) |
| (c) AVKHERD | (hereinafter referred to as Peptide 3) |
| (d) VYNGTSKYAVGGSGRR | (hereinafter referred to as Peptide 4) |
| (e) TYTGTTAYTASARR | (hereinafter referred to as Peptide 5) |
| (f) VYNGECRYSRNAVPNLR | (hereinafter referred to as Peptide 6) |
| (g) GSLAARVVKQ | (hereinafter referred to as Peptide 7) |
| (h) AHLAAAHARH | (hereinafter referred to as Peptide 8) |
| (i) VLPVQPTGDRH | (hereinafter referred to as Peptide 9) |
| (j) IQSLSPTHVI | (hereinafter referred to as Peptide 10) |
| (k) VHVSGNQHTL | (hereinafter referred to as Peptide 11) |
| (l) VHKDSI | (hereinafter referred to as Peptide 12) |
| (m) VPSHTL | (hereinafter referred to as Peptide 13) |
| (n) ESALLNTS | (hereinafter referred to as Peptide 14) |
| (o) VSALDNTA | (hereinafter referred to as Peptide 15) |
| (p) IKADAIH | (hereinafter referred to as Peptide 16) |
| (q) VKAETIT | (hereinafter referred to as Peptide 17) |
| (r) GSGRRGDMGSLAARVVKQLP | (hereinafter referred to as Peptide 18) |
| (s) NLRGDLQVLAQKVARTLP | (hereinafter referred to as Peptide 19) |
| (t) ASARRGDLAHLAAAHAR | (hereinafter referred to as Peptide 20) |
| (u) ARRGDLAHLAAAHARHLP | (hereinafter referred to as Peptide 21) |
| (v) VPNLRGDLQVLAQKVARTLP | (hereinafter referred to as Peptide 22) |
| (w) VLPVQPTGDRHKQPLI | (hereinafter referred to as Peptide 23) |

fragments thereof containing at least 6 amino acids and derivatives of said peptides or fragments.

Amino acids forming the components of the peptides and polypeptides referred to herein are each symbolised a single letter as follows:-

A alanine
C cysteine
D aspartic acid
E glutamic acid
F phenylalanine
G glycine
H histidine
I isoleucine
K lysine
L leucine
M methionine
N asparagine
P proline
Q glutamine
R arginine
S serine
T threonine
V valine
W tryptophan
Y tyrosine

The term 'fragment' as used herein refers to any peptide containing at least 6 amino acids, the sequence of the fragments being found within the sequence of the peptides defined above (ie. comprising a partial sequence of said peptides).

Of the peptides defined above those referred to as Peptides 1 to 9 and fragments thereof are preferred.

The peptides of the invention may be used in their own right as antigens for use in vaccination against FMDV. However when used as antigen it is preferred that the peptides of the invention be coupled to a suitable carrier, preferably a polypeptide, for example bovine serum albumin (BSA) or keyhole limpet haemocyanin (KLH).

The invention thus provides, in a further aspect, an antigen to FMDV characterised in that it comprises a peptide as defined hereinabove coupled to a carrier therefor.

The peptides of the invention are also useful in confering advantageous properties to larger peptides and polypeptides. Thus the peptides of the invention may comprise a sequence within a larger peptide or polypeptide. Such larger peptides may comprise only one or a few additional amino acid residues or may contain many more amino acid residues. Such larger peptides or polypeptides may comprise for example 2 or more peptides or polypeptides of the invention linked either directly or through one or more additional amino acid residues. Such larger peptides or polypeptides may comprise all or part of a capsid protein from FMDV, in particular VP1 either alone or as part of a larger protein additionally containing, for example, VP2, VP3, VP4 or p88. Advantageously the polypeptide will comprise all or part of a VP1 protein or a polypeptide similar thereto and processing similar antigenic characterisitics.
The invention thus provides in a further aspect a peptide or polypeptide characterised in that it is immunogenic for FMDV and comprises or includes as a partial sequence a peptide as defined hereinabove or a fragment thereof as defined above.

The peptides and polypeptides of the invention may be prepared by any method known in the art for the preparation of peptides or polypeptides.

Thus in a first method the peptides and polypeptides may be prepared synthetically by known techniques from individual amino acids, from linking of smaller peptides or a combination thereof.

In an second method the peptides and polypeptides may be obtained by cleavage, and optionally subsequent modification, of larger polypeptides or proteins.

In a further method the peptides and polypeptides of the invention may be prepared by the technique of genetic engineering as described for example with particular respect to FMDV antigens in European Patent Application Publication No. 0048455, which is incorporated herein by reference.

Thus the invention provides in a further aspect a method for the preparation of a peptide or polypeptide of the invention which method comprises:-

(a) transforming a host cell with recombinant DNA, said recombinant DNA comprising (1) a doubled stranded DNA sequence coding for or including a sequence for coding for a peptide of the invention (2) a cloning vehicle DNA sequence and (3) initiator and terminator sequences for expression of DNA;

(b) culturing the host cell under conditions suitable for expression of FMD peptide.

(c) harvesting the cell culture, and

(d) separating said peptide

There is further provided a DNA molecule comprising a nucleotide sequence for the peptides of the invention or a DNA molecule coding for a polypeptide which polypeptide includes a peptide of the invention.

There is additionally provided a recombinant DNA molecule comprising an operon having initiator sequences as hereinafter defined, and a nucleotide sequence substantially coding for a peptide of the invention or a nucleotide sequence including such a sequence, the nucleotide sequence being located between the initiator sequences and the terminator sequences of the operon.

The invention also provides a recombinant DNA cloning vehicle capable of expressing all or part of a peptide of the invention comprising an operon having initiator sequences and terminator sequences, and a nucleotide sequence substantially coding for said peptide the nucleotide sequence being located between the initiator sequences and terminator sequences of the operon.

In a further aspect of the invention there is provided a host cell containing a recombinant DNA cloning vehicle and/or a recombinant DNA molecule as defined above.

The invention also provides an antigen for stimulating the production of antibodies against FMDV in a mammal comprising a peptide of the invention prepared by the expression of a DNA molecule as hereinbefore defined and produced by a host cell transformed with a recombinat DNA cloning vehicle as hereinbefore defined and produced by a host cell transformed with a recombinant DNA cloning vehicle as hereinbefore defined.

The invention further provides a method of preparing a DNA molecule substantially coding for a peptide of the invention comprising:

(a) isolating FMDV single stranded RNA;

(b) preparing a first single strand of DNA complementary to the single strand of FMDV RNA;

(c) preparing a second single strand of DNA complementary to and hydrogen bonded to the first DNA strand so as to produce a double strand of DNA.

The double strand of DNA so produced can be inserted into a cloning vehicle following digestion of the cloning vehicle with a restriction endonuclease, free ends of the cloning vehicle being joined to the DNA molecule so as to form a recombinant cloning vehicle. This in turn can be inserted into a suitable host cell by for example transformation.

As used herein the terms listed below have the following meanings:-

Nucleotide: a unit of DNA or RNA comprising a sugar moiety ( pentose ), a phosphate and a nitrogenous heterocyclic base. The base is joined to the sugar moiety via the glycosidic carbon ( 1' carbon of the pentose ) and the base characterizes the nucleotide. The four DNA bases are adenine ( A ), guanine ( G ), cytosine ( C ) and thymine ( T ). The four RNA bases are A[ G[ C and uracil ( U ).

Recombinant DNA :- a hybrid double stranded DNA sequence comprising at least two double stranded DNA nucleotide sequences, the first sequence not being found together in nature with the second sequence.

Cloning Vehicle :- non-chromosomal double stranded DNA capable of replicating when placed within a unicellular micro-organism.

Plasmid :- a cloning vehicle derived from viruses or bacteria.

Structural Gene :- a sequence of DNA nucleotides which codes for a sequence of amino acids characteristic of a specific polypeptide.

Initiator Sequences :- sequences of DNA nucleotides which control the initiation of transcription or translation.

Terminator Sequences :- sequences of DNA nucleotides at which transcription or translation ceases.

Transcription :- the process whereby RNA polymerase is caused to move along the DNA sequence forming messenger RNA.

Translation :- the process of producing a polypeptide from messenger RNA.

Operon :- a structural gene(s) coding for polypeptide expression which is preceded by initiator sequences and succeeded by terminator sequences.

Expression :- the process involved in producing a polypeptide from a structural gene.

The invention will be further described by way of reference to the accompanying drawings in which:-

Figure 1 shows physical maps and sequencing strategy used;
Figure 2 shows the nucleotide and predicted amino acid sequence for VP1 regions of 3 serotypes of FMDV;
Figure 3 shows a comparison of the amino acid sequencesof Figure 2 with 3 published sequences for VP1; and
Figure 4 shows the variability of three sequences of VP1.

In Figure 1 the fragments used for sequencing are indicated by ●⟶ where ● indicates the labelled end. Restriction enzyme sites used to generate the labelled end are shown. The abbreviations for the sites are:- A, Ava II; B, Bam HI; E EcoRI; G, Bgl II; H, Hind III; M Sma I; P, Pst I; (P), expected Pst I site which was lost presumably by slight exonuclease contamination; R, Rsa I, S, Sst I. The polypeptides encoded by each region are shown.

In Figure 2 the nucleotide and predicted amino acid sequences for the VP1 regions of (a) $A_{24}$ Cruzeiro, (b) $C_3$ Indaial, (c) $O_1$BFS are shown:-
(a) The first eleven codons of VPI in $A_{24}$ are presumed missing.
(b) * The C was read as a T in the plasmid pF$C_3$301 but is presumed to be a C in the parental plasmid pF$C_3$r3.
(c) The underlined sequence was derived from both plasmids pF$O_1$t351 and pF$O_1$r2.
† The C was derived from pF$O_1$r2 and was replaced by a T in pF$O_1$t351, which would predict a methionine residue.

In Figure 3 a comparison of the amino acid sequences between (a) $A_{12}$, $A_{24}$ and $A_{10}$, (b) $A_{10}$, $C_3$ and $O_1$BFS, (c) $O_1$BFS and $O_1$K is shown. The amino acids were aligned for maximum homology with the minimum introduction of gaps, each indicated by a -. Each position in VPI is numbered from 1 to 216, including the gaps. All difference within a comparison group are boxed. † The threonine residue was predicted in pF$O_1$r2 but a methionine was predicted in pF$O_1$t351.

In Figure 4 the variability of $A_{10}$61, $C_3$ Indaial and $O_1$BFS plotted with (a-c) hydrophilicity of $A_{10}$, $C_3$ or $O_1$ or with (d) linear representations of peptides tested for immunogenicity. Each type of plot is generated by combining the scores of five positions in the sequence, each point representing the middle (i.e. third) position of each group of five. The variability plot (———) uses a score of 0 for every identical comparison for each position and a score of 1 for every different comparison for each position. This gives a maximum score of 3 for each position and 15 for each group of 5 positions. The hydrophilicity plots (......) using the scoring system of Hopp and Woods (P.N.A.S., 1981, 78, 3824-3828) for each amino acid, the scores ranging from -3.4 to +3.0. This gives a maximum score of 15 and a minimum score of -17 for each group of 5 positions. In order to align the two plots gaps were introduced in the hydrophilicity plots corresponding to gaps in the appropriate sequence in Fig. 3 which were used in the variability plot. The major hydrophilicity peaks are numbered 1-5

(d) ——— immunogenic peptide, -----non-immunogenic peptide. (A,B) peptides generated by trypsin; (C,D) peptides generated by mouse submaxiliary gland protease; (E-H) peptides generated by cyanogen bromide (Strohmaier et al., J. Gen. Virol., (1982) 59, 295-306); (I-P) peptides produced by chemical synthesis (Bittle et al., 1982).

Further characteristics and features of the invention are described in the following Examples which are presented by way of illustration only and are not to be considered as limiting the scope of the present invention in any way.

EXAMPLE 1

(a) Preparation of FMDV RNA

Approximately 10 ml of a recent harvest of FMDV type $A_{24}$ Cruzeiro in Eagle's medium were added to each of ten Roux bottles containing monolayers of approximately $10^8$ $BHK_{21}$ cells. After gentle shaking for 30 min the medium was decanted and 20 ml fresh medium added to each bottle. After the virus infection had destroyed the cell monolayers ( 3-4h ), the medium was decanted and the cell debris was removed by centrifugation at 12,000xg for 15 min at $4^oC$. The virus was then pelleted by spinning at 90,000xg for 1hr at $4^oC$. The virus pellet was resuspended in 2 ml of TNE buffer ( 10mM Tris-HCl ( pH 7.5) 150mM NaCl and 1mM EDTA ( ethylenediamino tetracetic acid ) and the suspension was cleared by centrifuging for 10 min at $20^oC$ at about 5,000xg. The cleared supernatant was made 1% w/v in sodium dodecyl sulphate ( SDS ) and loaded onto a preformed gradient ( sucrose 15-45% w/v ) in TN buffer ( 100mM Tris-HCl pH 7.6, 100mM NaCl ) and spun at 100,000xg for 2hr at $10^oC$. Fractions ( 1 ml ) were monitored at 260nm and the fractions containing virus were extracted once with an equal volume of phenol:chloroform ( 1:1 ) and the aqueous phase precipitated by the addition of 2 volumes ethanol, incubating overnight at $-20^oC$. The RNA precipitate was pelleted by spinning at 5,000xg for 30min at $4^oC$. The supernatant was discarded, the pellet drained and then dissolved in 0.5 ml TNES buffer ( 10mM Tris-HCl pH 7.6, 150mM NaCl, 1mM EDTA, and 0.2% w/v SDS ). This solution was loaded onto a

preformed sucrose gradient ( 5-25% ) in TNES and centrifuged at 200,000xg for 3.5hr at 20°C. Fractions ( 0.5 ml )containing RNA sedimenting at 35S were pooled, phenol-chloroform extracted once, ethanol precipitated and redissolved as described for virus above. The resulting solution was ethanol precipitated and finally dissolved in 0.05 ml of double distilled water.

By a similar technique FMDV RNA was also obtained from FMDV types $C_3$ Indaial and $O_1$BFS.

(b) Synthesis of double stranded complementary DNA ( DScDNA )

FMDV RNA ( 10 ug ) and either oligo-dT$_{( 12-18 )}$primer ( 0.5 ug; obtained from Collaborative Research ) or a calf thymus DNA DNAaseI digest were incubated at 30°C for 2 hr in a final volume of 100 ul containing 0.4mM dithiothreitol, 8mM $MgCl_2$, 50mM Tris-HCl pH 8.1 148mM NaCl, 0.2mM dATP ( 2.5Ci/m mole / $^{32}P$ /-dATP; obtained from Radiochemical Centre, Amersham ), 0.2mM dTTP, 0.2mM dCTP, 0.2mM dGTP, 4mM $Na_4P_2O_7$ and 28 units AMV reverse transcriptase ( supplied by Dr. J. Beard, Life Sciences Inc., Florida ). The reaction was stopped by adding EDTA ( 20mM ) and SDS ( 0.2% w/v ). A sample ( 2% ) of this terminated reaction mixture was spotted on 2.5cm DE81 paper discs ( obtained from Whatman ). These were washed extensively in 5% (w/v) $Na_2HPO_4$, followed by a brief wash ( 5 min ) in distilled water before drying and scintillation counting. From this procedure, a total yield of approximately 2 ug of cDNA was calculated. The remainder of the reaction mixture was phenol-chloroform extracted and ethanol precipitated as described above except that sodium acetate was added to 0.2M before addition of the ethanol. The desiccated pellet was dissolved in 100 ul 0.1M NaOH and incubated at 70°C for 20 min to remove the template i.e. FMDV RNA. The solution was neutralised with acetic acid and the cDNA resolved from degraded RNA by passing through a column ( 100 x 15mm ) of bead-f phenol-chloroform. The desiccated pellet was resuspended to a final volume of 90 ul in 50mM Tris-HCl pH 8.3 20mM dithiothreitol, 10mM $MgCl_2$, 0.4mM dCTP, 0.4mM dGTP, 0.4mM dATP ( 5.5Ci/m mole / $^{32}P$ /-dATP; Radiochemical Centre, Amersham ), 0.4mM dTTP, and 36 units AMV reverse transcriptase. After 4 hr incubation at 45°C the reaction was stopped by the addition of EDTA ( 20mM ) and SDS ( 0.2% w/v ). The mixture was phenol-chloroform extracted, NaCl was added to 0.2M and the mixture precipitated with 2 volumes of ethanol overnight at -20°C. The desiccated pellet was resuspended in 200 ul of 50mM NaCl, 0.1% w/v SDS and passed through a Sephadex G100 column as described above. The excluded peak was ethanol precipitated in the presence of 200 ug glycogen carrier. DE81 paper disc analysis, as described above, indicated the second strand synthesis was about 60% of the maximum theoretical yield. The desiccated pellet from ethanol precipitation was resuspended in a final volume of 200 ul S1 buffer ( 25mM sodium acetate ( pH 4.6 ), 150mM NaCl, and 1mM $ZnSO_4$ ). A 28 ul sample of this ( sample A ) was stored at -20°C while the remainder ( sample B ) was incubated with 5 units $S_1$ nuclease ( obtained from Sigma ) for 30 min at 37°C. The reaction was stopped by extraction with phenol-chloroform and the aqueous phase precipitated with ethanol. The desiccated pellet was resuspended in 20 ul $H_2O$. To check the effectiveness of the S1 nuclease treatment in removing the loops in the doublestranded cDNA, a small amount ( 2% ) of samples A and B was heated in S1 buffer at 100°C for 6 min, cooled to 60°C and 3 units of S1 added at varying times ( 0, 0.25, 1 and 14 hr ) followed by incubation at 37°C for 30 min. Resistance to S1 was assayed by DE81 paper disc binding as described above. The results showed that greater than 53% of sample A was resistant to S1 digestion compared with less than 2% of sample B when both were incubated with S1 immediately upon cooling to 60°C. If S1 was added 0.25h after the samples were placed at 60°C these figures rose to 81% and 15%, respectively. Hence the initial S1 treatment had effectively cleaved the loops present after second strand synthesis.

(c) Homopolymer tailing of plasmid and double stranded cDNA ( ds cDNA )

( i ) dG-tailing of the vector. Approximately 7 ug of the vector, plasmid pAT153, were digested with Pst 1 endonuclease under conditions recommended by the enzyme suppliers ( Boehringer ). The reaction was stopped by the addition of sodium acetate to 0.3M and ethanol precipitated. The desiccated pellet was resuspended in 100 ul of dG-tailing buffer ( 100mM sodium cacodylate-HCl pH 7.1), 5mM $MgCl_2$, 50ug/ml bovine albumin and 1mM dGTP ( 200mCi/8$^3$-H /-dGTP/m mole ). To this, 2 ul ( 38 units ) of terminal transferase ( TT) were added and the reaction incubated at 37°C. Samples were removed after 2.5, 5 and 10 min. In each case the reaction was stopped by chilling on ice and adding EDTA to 20mM. Analysis by trichloroacetic acid precipitation of a 5 ul sample from each showed that no further incorporation of $^3$H dGMP occurred after 2.5 min and that the average length of the homopolymer tail by this time was about 25 nucleotides. The remainder of the 2.5 min sample was diluted to 100 ul with TE buffer ( 10mM Tris-HCl pH 8.0, 1mM EDTA) and extracted once with an equal volume of TE-saturated phenol. The aqueous phase was extracted four times with ether; sodium acetate was added to 0.3M and the sample ethanol precipitated. The desiccated pellet was resuspended in 40 ul $H_2O$ and stored at -10°C. The concentration of DNA in this final solution was estimated to be 15ug/ml.

( II ) dC-tailing of dscDNA: Approximately 0.4ug of dscDNA in dC-tailing buffer ( 100mM sodium cacodylate HCl ( pH 7.1 ), 1mM $CoCl_2$, 0.1mM DTT, 50ug/ml bovine serum albumin 0.5mM $^3H$ dCTP ( 600mCi/ 5-$^3H$ /-CTP/m mole ) was incubated with 1 ul TT ( 19 units ). Aliquots were removed after 2 and 5 min and assayed for incorporation by TCA precipitation of a 5 ul sample of each. This indicated the average length of the homopolymer tail to be about 70 nucleotides after 2 min and 160 nucleotides after 5 min. These two aliquots were pooled, phenol extracted once, ether extracted four times, ethanol precipitated, redissolved in 50 ul $H_2O$ and stored at -10°C.

(d) Transformation with annealed plasmid and dscDNA

Approximately 0.12ug of dG-tailed plasmid pAT153 ( 0.05pmoles ) and 0.1ug dC-tailed dscDNA ( 0.2-0.4pmoles ) were incubated at 65°C for 0.5h in 100ul TNE Buffer ( 10mM Tris-Cl pH 8.0, 200mM NaCl, 1mM EDTA ). The temperature of the incubation was steadily dropped to 20°C over a 4 hour period and then rapidly brought to 0°C. The solution was diluted to 200ul containing, finally, 15mM Tris-HCl pH 7.5, 100mM NaCl, 10mM $MgCl_2$, 10mM $CaCl_2$, 0.5mM EDTA and stored at 0°C for about 3 hours.

A transformation competent culture of E.coli HB101 was prepared by inoculating 1ml of a stationary phase culture into 65ml L-broth ( 1% Difco Bacto Tryptone, 0.5% Difco Bacto Yeast Extract and 0.5% NaCl, pH 7.2 ) and shaking at 37°C for about 3h after which time the $A_{650}$ was 0.4. The cells were pelleted by spinning at 3,000xg for 5 min at 4°C. The cells were resuspended in 25ml 0.1M $MgCl_2$ ( 0°C ) and repelleted. The pellet was resuspended in 2ml 0.1M $CaCl_2$ ( 0°C) and left for 30min at 0°C. Approximately 0.2ml of such transformation-competent cells were gently mixed with 0.1ml of annealed pAT153/dscDNA and left for a further 30min at 0°C, followed by 2min at 42°C, and a final 30min at 0°C. To this was added 1ml L-broth and the transformation mix then incubated at 37°C for 25min. Ten 0.1ml samples of this were spread onto L-Tc plates ( L-broth plus 1.5% agar and 15ug/ml tetracycline ( obtained from Sigma ) ) and incubated overnight at 37°C. From these ten plates 150 tetracycline resistant colonies were obtained of which about 87% were sensitive to ampicillin ( 100ug/ml; Sigma ) suggesting insertion of cDNA at the Pst 1 site of pAT153.

(e) Large scale recombinant plasmid preparation

The transformants containing the plasmids were grown in bulk by inoculating 10ml stationary culture into 500ml L-broth and shaking at 37°C for about 4h until $A_{590}$ reached about 1.0. Chloramphenicol ( obtained from Sigma) was then added to 0.1mg/ml and the cultures shaken at 37°C overnight. After this amplification step the cells were pelleted by centrifuging at 5000xg for 5min at 4°C. The supernatant was disinfected and discarded, and the cell pellet resuspended in 12ml R buffer ( 50mM Tris-HCl ( pH 8,0 ), 40mM EDTA, 25% sucrose ). Lysosyme and EDTA were then added to 1.4mg/ml and 60mM respectively and the suspension left on ice for 5min. To 16ml of this were added 30ml Triton mix ( 0.1% Triton X-100, 62.5mM EDTA, 50mM Tris-HCl pH 8.0 ) and the mixture left on ice about 10min or until viscous. The mixture was then cleared by spinning at 48,000xg for 15min at 4°C. The resulting supernatant was carefully decanted and 0.95g CsCl and 0.1ml of a 10mg/ml solution of ethidium bromide added per ml. This was spun at 12000xg in a Beckman 50Ti rotor at 20°C for 40h. The plasmid band was visualised by long wave U.V. fluoresence and removed by syringe by piercing the side of the tube. The plasmid DNA was spun to equilibrium in a second CsCl/ethidium bromide gradient. The resulting band was extracted 4 times with propan-2-ol saturated with NaCl and $H_2O$, and then ethanol precipitated, redissolved in TE and reprecipitated. The final desiccated pellet was resuspended in 200ul TE buffer and stored at 4°C.

(f) Screening of Recombinants

The transformed cells obtained above were screened by the colony hybridisation of Hanahan and Meselon (Gene 10, 63-67(1980)). The $C_3$ and $A_{12}$ recombinant plasmid transformed cells were probed with a Pvu II fragment of $pFA_{10}t76$ (Boothroyd et al Gene 17, 153-161 (1982)) which contains DNA sequences derived from $A_{10}61$, covering most of both VP3 and VP1. The $O_1$ transformed cells were probed with a small RsaI/HinfI fragment of $pFO_1t351$ which was derived from the plasmid $pFO_1t251$ (Boothroyd et al Nature, 290, 800-802 (1981)) and comprises mainly the 3' end of VP1.

The positive colonies were further screened by restriction mapping of their plasmid DNA. These maps were aligned with a linear map of the protein coding units of the FMDV genome, by analogy with the cDNA sequences of $A_{10}61$ (Boothroyd et al Nature, 290, 800-802 (1981)), of $A_{12}119$ (Kleid et. al, Science, 214, 1125-1129 (1981)) and of $O_1K$ (Kurz et. al., Nucleic Acids Research, 9, 1919-1931 (1981)). In some cases, Southern blotting of appropriate restriction fragments was necessary to resolve ambiguities.

Sequencing and Analysis of Sequence Data

DNA was sequenced by the method of Maxam and Gilbert (Methods in Enzymology Vol. 65, 499-560, Grossman, L. and Moldave, K. (Eds), Academic Press, New York) as described by Boothroyd et. al. (Gene, 17, 153-161 (1982)).

An Apple II microcomputer was used to search DNA sequences for restriction sites and to predict the corresponding protein sequences, using a modification of a programme written by Larson and Messing (Nucleic Acids Research, 10, 39-49 (1982)). It was also used to make hydrophilicity plots using the programme in Kyte and Doolittle (J.mol. Biol., 157, 105-132 (1982)). A PDP II minicomputer was used to determine dinucleotide frequencies and codon usage, using programme written by Staden (Nucleic Acids Research 4, 4037-4051 (1977) and Nucleic Acids Research, 8, 3673-3694 (1980)).

The VP1 coding sequences for the three serotypes $A_{24}$, $C_3$ and O,BFS were thus determined and the predicted amino acid sequences were compared with three known VP1 sequences $A_{10}$ (Boothroyd et. al. Gene 17, 153-161 (1982)), $A_{12}$ (Kleid et. al. Science 214, 1125-1129 (1981)) and $O_1K$ (Kurz et. al., Nucleic Acids Research, 9 1919-1931 (1981)). The results are shown in Figures 2 and 3.

Comparison of Amino Acid Sequences

Amino acid sequences for VP1 from the three serotypes $A_{24}$, $C_3$ and $O_1BFS$ were predicted from the nucleotide sequences determined above and are shown in Figure 2. The sequences were compared to known amino acid sequences for VP1 of three other strains.

The sequences were aligned to give maximum homology which necessitated the introduction of gaps (indicated by dashes) but these were kept to a minimum. They are arranged to enable comparisons to be made between (b) different serotypes ($A_{10}$, $C_3$ $O_1BFS$), (a) different subtypes of the same serotype ($A_{10}$, $A_{12}$, $A_{24}$) and (b) different isolates of the same subtype ($O_1BFS$, $O_1K$). Some of the flanking sequence is included, but only VP1 is numbered. In order to maintain consistency between different sequences, the gaps have been included in the numbering system. The results are shown in Figure 3.

Determination of Immunogenic Amino Acid Sequences

Comparison of VP1 sequences form different strains of FMDV revealed a number of

variable regions interspersed by conserved regions. It is thought likely that immunogenic regions are under strong selection pressure to vary in order to evade the host immune system. Therefore some of the variable regions are likely to be immunogenic. Since immunogenic regions are almost certainly located on the surface of the virus they are likely to be themselves hydrophilic or situated near to hydrophilic regions. Figure 4 shows plots of predicted hydrophilicity VP1 from each of the 3 serotypes studied, super-imposed on a plot of variability of VP1 from $A_{10}$, $C_3$, $O_1$. This identified those variable regions which overlapped hydrophilic regions and are therefore more likely to be immunogenic.

EXAMPLE 2. Preparation of Peptide Fragments.

(a) A peptide fragment corresponding to the region 143-162 of $A_{24}$ Cruzeiro $VP_1$, which has the structure GSGRRGDMGSLAARVVKQLP, was synthesized by the solid phase method (Marglin,A, Merrifield, R.B. Ann. Review Biochem. 39, p.841-866, 1970) on chloromethyl polystyrene resin using a custom built automatic synthesiser.

tBoc-pro-Resin was purchased from Sigma (Poole, England). All the coupling reactions used a 3-fold excess of tBoc amino acid and dicyclohexyl carbodinimide and were tested by ninhydrin (11). Where the reaction was less than 99% complete, the coupling procedure was repeated.

The peptide was removed from the support and deprotected with 20ml hydrogen fluoride plus 5ml anisole as scavenger and 2.5ml ethyl methyl sulphide, (the latter to protect the methionine) at 4°C for 30 minutes. The hydrogen fluoride was removed with a stream of nitrogen. The peptide and resin were then dried under vacuum to remove all traces of HF, then washed with ether to remove the anisole and remaining ethyl methyl sulphide. The resin was then air dried to remove the ether.

The peptide was extracted with 10% acetic acid. The purity was assayed by amino acid analysis and by HPLC on a reverse phase column of Zorbax C8 (DuPont) using 0.1% trifluoroacetic acid and an acetonitrile gradient of 0-60%. The peptide was desalted on Sephadex G10 and evaporated to dryness to give the required product.

(b)Similarly prepared was a peptide fragment corresponding to region 143-162 of $O_1$BFS 1860 $VP_1$ and having to sequence:-
NLRGDLQVLAQKVARTLP.

## CLAIMS

1. A peptide characterised in that it comprises the amino acid sequence of an antigenic site of a $VP_1$ protein selected from serotypes $A_{24}$ Cruzeiro, $C_3$ Indaial or $O_1$ BFS, fragments thereof and derivatives thereof.

2. A peptide as claimed in claim 1 characterised in that it corresponds to all or part of a region of $VP_1$, selected from 42-61; 79-85; 95-102; 129-160 (or 162); 170-176; 193-204 (or 206).

3. A peptide as claimed in either claim 1 or claim 2 characterised in that it corresponds to a region of $VP_1$ selected from 79-85; 129-160 (or 162) and 193-204 (or 209).

4. A peptide as claimed in any one of claims 1 to 3 characterised in that it corresponds to a region of $VP_1$ selected from 42-51; 56-61; 129-147; 151-160 and 143-162.

5. A peptide as claimed in any one of claims 1 to 4 and selected from:-

(a) VVRHEGN
(b) AVTHTGK
(c) AVKHERD
(d) VYNGTSKYAVGGSGRR
(e) TYTGTTAYTASSARR
(f) VYNGECRYSRNAVPNLR
(g) GSLAARVVKQ
(h) AHLAAAHARH
(i) VLPVQPTGDRH
(j) IQSLSPTHVI
(k) VHVSGNQHTL
(l) VHKDSI
(m) VPSHTL
(n) ESALLNTS
(q) VKAETIT
(r) GSGRRGDMGSLAARVVKQLP
(s) NLRGDLQVLAQKVARTLP
(t) ASARRGDLAHLAAAHAR
(u) ARRGDLAHLAAAHARHLP
(v) VPNLRGDLQVLAQKVARTLP
(w) VLPVQPTGDRHKQPLI

(o) VSALDNTA

(p) IKADAIH

6. The peptide having the sequence GSGRRGDMGSLAARVVKQLP.

7. The peptide having the sequence NLRGDLQVLAQKVARTLP.

8. A protein having the structure of $VP_1$ of FMDV serotypes $A_{24}$ Cruzeiro, $C_3$ Indaial or $O_1$ BFS as shown in Figure 3.

9. An immunogen comrising a peptide as defined in any one of claims 1 to 8 linked to a carrier.

10. A vaccine comprising a peptide as defined in any one of claims 1 to 8 or an immunogen as defined in claim 9 together with a pharmaceutically acceptable vehicle therefor.

11. A method of preparing a peptide or protein as defined in any one of claims 1 to 8 comprising:-

A. Synthetic preparation by known techniques from individual amino acids, from linking of smaller peptides or a combination thereof;

B. Cleavage, and, optionally subsequent modification, of larger polypeptides or proteins; or

C. (a) transforming a host cell with recombinant DNA, said recombinant DNA comprising (1) a doubled stranded DNA sequence coding for or including a sequence coding for a protein or for a peptide as defined in any one of claims 1 to 8 (2) a cloning vehicle DNA sequence and (3) initiator and terminator sequences for expression of DNA;

(b) culturing the host cell under conditions suitable for expression of FMD peptide;

(c) harvesting the cell culture, and

(d) separating said peptide

12. A DNA molecule comprising a nucleotide sequence for a protein or peptide as defined in any one of claims 1 to 8 or a DNA molecule coding for a polypeptide which polypeptide includes a peptide or protein as defined in any one of claims 1 to 8.

13. A recombinant DNA molecule comprising an operon having initiator sequences as herein defined, and a nucleotide sequence substantially coding for protein or peptide as defined in any one of claims 1 to 8 or a nucleotide sequence including such a sequence, the nucleotide sequence being located between the initiator sequences and the terminator sequences of the operon.

14. A recombinant DNA cloning vehicle capable of expressing all or part of a protein or peptide as defined in any one of claims 1 to 8 comprising an operon having initiator sequences and terminator sequences, and a nucleotide sequence substantially coding for said peptide or protein the nucleotide sequence being located between the initiator sequences and terminator sequences of the operon.

15. A host cell containing a recombinant DNA cloning vehicle and/or a recombinant DNA molecule as defined in any one of claims 12 to 14.

16. An antigen for stimulating the production of antibodies against FMDV in a mammal comprising a protein or a peptide as defined in any one of claims 1 to 8 prepared by the expression of a DNA molecule as hereinbefore defined and produced by a host cell transformed with a recombinat DNA cloning vehicle as hereinbefore defined and produced by a host cell transformed with a recombinant DNA cloning vehicle as hereinbefore defined.

17. A method of preparing a DNA molecule substantially coding for a peptide as defined in any one of claims 1 to 8 comprising

(a) isolating FMDV single stranded RNA;
(b) preparing a first single strand of DNA complementary to the single strand of FMDV RNA; and
(c) preparing a second single strand of DNA complementary to and hydrogen bonded to the first DNA strand so as to produce a double strand of DNA.

18. A peptide or protein as defined in any one of claims 1 to 8 for use in therapy.

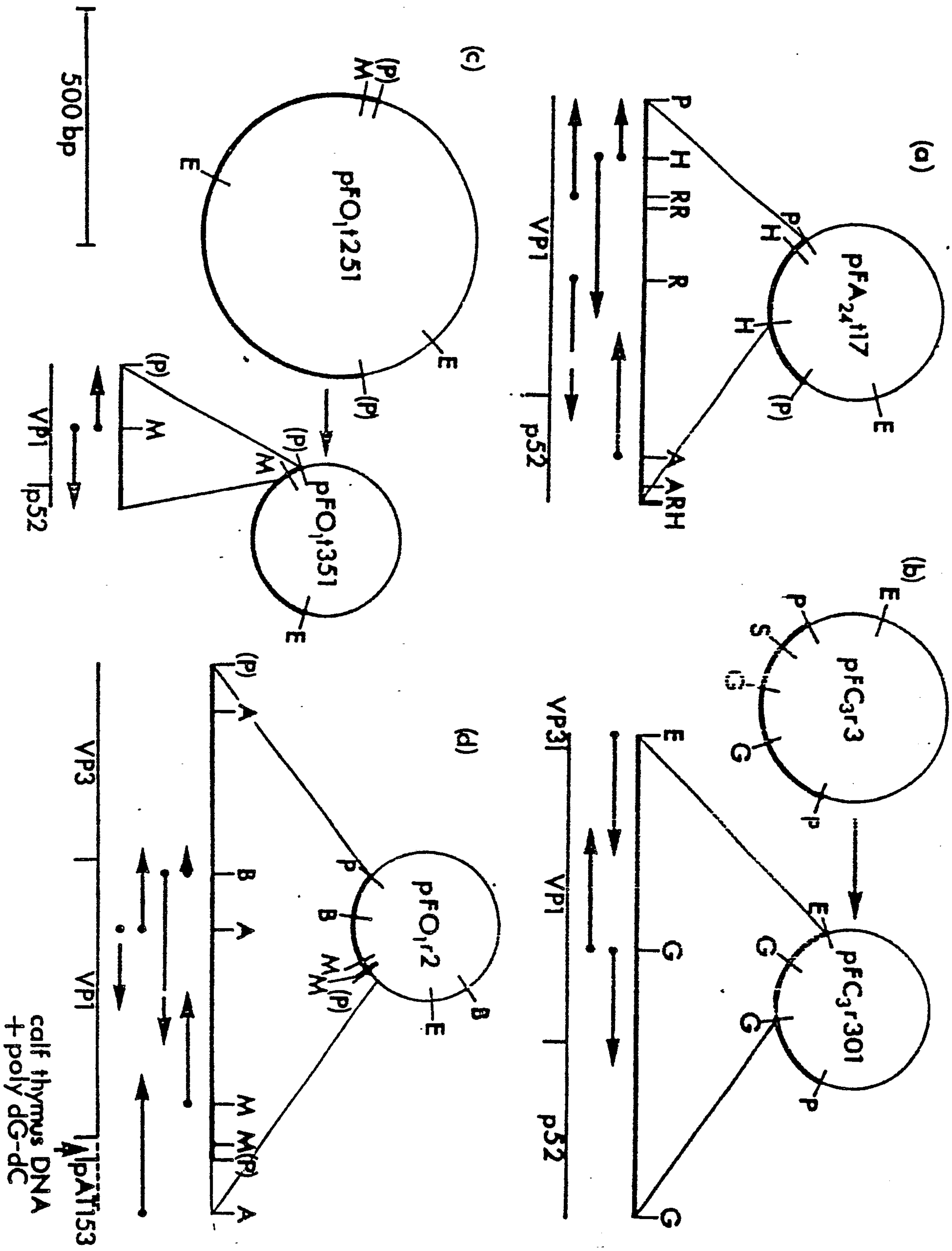
(a)
pFA24t17
(b)
pFC3r3
pFC3r301
(c)
pFO1t251
pFO1t351
(d)
pFO1r2
VP1
VP3
p52
500bp
calf thymus DNA
+ poly dG-dC
pAT153

FIG. 1

(a)

```
VP1→
ACC ACC ACC GTG GAG AAC TAC GGC GGT GAG ACA CAA ATC CAG AGA CGT CAC CAC ACG GAC   60
THR THR THR VAL GLU ASN TYR GLY GLY GLU THR GLN ILE GLN ARG ARG HIS HIS THR ASP

ATT GGT TTC ATC ATG GAC AGA TTT GTG AAG ATC CAA AGC TTG AGC CCA ACA CAT GTC ATT   120
ILE GLY PHE ILE MET ASP ARG PHE VAL LYS ILE GLN SER LEU SER PRO THR HIS VAL ILE

GAC CTC ATG CAG ACT CAC CAA CAC GGT CTG GTG GGT GCC TTG CTG CGT GCA ACC ACG TAC   180
ASP LEU MET GLN THR HIS GLN HIS GLY LEU VAL GLY ALA LEU LEU ARG ALA THR THR TYR

TAC TTT TCT GAC CTG GAA ATT GTT GTA CGG CAC GAA GGC AAT CTG ACC TGG GTG CCC AAC   240
TYR PHE SER ASP LEU GLU ILE VAL VAL ARG HIS GLU GLY ASN LEU THR TRP VAL PRO ASN

GGC GCC CCT GAA TCA GCC CTG TTG AAC ACC AGC AAC CCC ACT GCC TAC AAC AAG GCA CCA   300
GLY ALA PRO GLU SER ALA LEU LEU ASN THR SER ASN PRO THR ALA TYR ASN LYS ALA PRO

TTC ACG AGA CTC GCT CTC CCC TAC ACT GCG CCG CAC CGT GTG CCG GCA ACA GTG TAC AAC   360
PHE THR ARG LEU ALA LEU PRO TYR THR ALA PRO HIS ARG VAL PRO ALA THR VAL TYR ASN

GGG ACG AGT AAG TAT GCT GTG GGT GGT TCA GGC AGA AGA GGC GAC ATG GGG TCT CTC GCG   420
GLY THR SER LYS TYR ALA VAL GLY GLY SER GLY ARG ARG GLY ASP MET GLY SER LEU ALA

GCG CGA GTC GTG AAA CAG CTT CCT GCT TCA TTT AAC TAC GGT GCA ATC AAG GCC GAC GCC   480
ALA ARG VAL VAL LYS GLN LEU PRO ALA SER PHE ASN TYR GLY ALA ILE LYS ALA ASP ALA

ATC CAC GAA CTT CTC GTG CGC ATG AAA CGG GCC GAG CTC TAC TGC CCC AGA CCG CTG TTG   540
ILE HIS GLU LEU LEU VAL ARG MET LYS ARG ALA GLU LEU TYR CYS PRO ARG PRO LEU LEU

GCA ATA GAG GTG TCT TCG CAA GAC AGG CAC AAG CAA AAG ATC ATT GCA CCA GCA AAG CAG   600
ALA ILE GLU VAL SER SER GLN ASP ARG HIS LYS GLN LYS ILE ILE ALA PRO ALA LYS GLN

←VP1 | p52→
CTT CTG|AAT TTT GAC CTG CTC AAG TTG GCC GGA GAC GTT GAG TCC AAC
LEU LEU|ASN PHE ASP LEU LEU LYS LEU ALA GLY ASP VAL GLU SER ASN
```

(b)

```
                                    ←VP3 | VP1→
CTC CGG CTA CCT GTG GAC GCT AGA CTG CAA|ACT ACG ACC ACT GGT GAA TCT GCC GAC CCC   60
LEU ARG LEU PRO VAL ASP ALA ARG LEU GLN|THR THR THR THR GLY GLU SER ALA ASP PRO

GTC ACC ACT ACC GTT GAG AAC TAC GGA GGA GAA ACA CAA ATC CAA CGT CGC CAC CAC ACT   120
VAL THR THR THR VAL GLU ASN TYR GLY GLY GLU THR GLN ILE GLN ARG ARG HIS HIS THR

GAC GTT GCC TTC GTT CTT GAC CGG TTT GTC AAG GTC CAT GTG TCG GGT AAC CAA CAT ACA   180
ASP VAL ALA PHE VAL LEU ASP ARG PHE VAL LYS VAL HIS VAL SER GLY ASN GLN HIS THR

CTG GAC GTT ATG CAG GTA CAC AAG GAC AGT ATT GTG GGC GCA CTC CTC CGC GCA GCC ACA   240
LEU ASP VAL MET GLN VAL HIS LYS ASP SER ILE VAL GLY ALA LEU LEU ARG ALA ALA THR

TAT TAC TTC TCT GAC TTG GAA ATA GCA GTG ACC CAC ACT GGG AAG CTC ACA TGG GTG CCC   300
TYR TYR PHE SER ASP LEU GLU ILE ALA VAL THR HIS THR GLY LYS LEU THR TRP VAL PRO

AAC GGC GCC CCA GTT TCT GCA CTT GAC AAC ACA GCC AAC CCC ACT GCC TAC CAC AAG GGA   360
ASN GLY ALA PRO VAL SER ALA LEU ASP ASN THR ALA ASN PRO THR ALA TYR HIS LYS GLY

CCG CTG ACT CGG CTG GCT CTC CCA TAC ACC GCA CCA CAC CGC GTG TTG GCC ACG ACG TAC   420
PRO LEU THR ARG LEU ALA LEU PRO TYR THR ALA PRO HIS ARG VAL LEU ALA THR THR TYR

ACC GGT ACA ACG GCC TAC ACT GCC AGT GCA CGT AGG GGA GAT CTA GCC CAC TTG GCG GCG   480
THR GLY THR THR ALA TYR THR ALA SER ALA ARG ARG GLY ASP LEU ALA HIS LEU ALA ALA

GCG CAC GCT CGG CAC CTG CCG ACG TCG TTC AAC TTT GGT GCA GTT AAA GCA GAG ACA ATT   540
ALA HIS ALA ARG HIS LEU PRO THR SER PHE ASN PHE GLY ALA VAL LYS ALA GLU THR ILE

ACA GAG CTG CTT GTG CGC ATG AAA CGT GCT GAA CTC TAC TGC CCC AGA CCG GTC CTT CCG   600
THR GLU LEU LEU VAL ARG MET LYS ARG ALA GLU LEU TYR CYS PRO ARG PRO VAL LEU PRO

                                                                        ←VP1
GTC CAA CCA ACG GGC GAC AGA CAC AAG CAA CCG CTC ATT GCG CCA GCG AAA CAA CTG CTG|
VAL GLN PRO THR GLY ASP ARG HIS LYS GLN PRO LEU ILE ALA PRO ALA LYS GLN LEU LEU|

|p52→
|AAC TTC GAC CTT CTC AAG TTG GCG GGA GAC GTC GAG TCC AAC
|ASN PHE ASP LEU LEU LYS LEU ALA GLY ASP VAL GLU SER ASN
```

(c)

```
                                    ←VP3 | VP1→
CTA AGG CTG CCG GTG GAC GCC CGT GCG GAA|ACC ACT TCT GCG GGC GAG TCA GCG GAT CCT   60
LEU ARG LEU PRO VAL ASP ALA ARG ALA GLU|THR THR SER ALA GLY GLU SER ALA ASP PRO

GTC ACC ACC ACT GTT GAA AAC TAC GGT GGC GAA ACA CAG ATC CAG AGG CGC CAA CAC ACG   120
VAL THR THR THR VAL GLU ASN TYR GLY GLY GLU THR GLN ILE GLN ARG ARG GLN HIS THR

GAC GTC TCG TTC ATT ATG GAC AGA TTT GTG AAA GTG ACA CCG CAA AAC CAA ATT AAC ATT   180
ASP VAL SER PHE ILE MET ASP ARG PHE VAL LYS VAL THR PRO GLN ASN GLN ILE ASN ILE

TTG GAC CTC ATG CAG GTT CCA TCA CAC ACT TTG GTG GGA GCG CTC CTA CGC GCG TCC ACT   240
LEU ASP LEU MET GLN VAL PRO SER HIS THR LEU VAL GLY ALA LEU LEU ARG ALA SER THR

TAC TAC TTC TCT GAC TTG GAG ATA GCA GTA AAA CAC GAG AGA GAC CTC ACC TGG GTT CCA   300
TYR TYR PHE SER ASP LEU GLU ILE ALA VAL LYS HIS GLU ARG ASP LEU THR TRP VAL PRO

AAT GGA GCG CCT GAA AAG GCG TTG GAC AAC ACC ACC AAC CCA ACT GCT TAC CAC AAG GCA   360
ASN GLY ALA PRO GLU LYS ALA LEU ASP ASN THR THR ASN PRO THR ALA TYR HIS LYS ALA

CCA CTC ACC CGG CTT GCC CTG CCC TAC ACT GCG CCC CAC CGC GTG TTG GCA ACC GTG TAC   420
PRO LEU THR ARG LEU ALA LEU PRO TYR THR ALA PRO HIS ARG VAL LEU ALA THR VAL TYR

AAC GGT GAG TGC AGG TAC AGC AGA AAT GCT GTG CCC AAC TTG AGA GGT GAC CTT CAA GTG   480
ASN GLY GLU CYS ARG TYR SER ARG ASN ALA VAL PRO ASN LEU ARG GLY ASP LEU GLN VAL

                                †
TTG GCT CAA AAG GTG GCA CGG ACG CTG CCT ACC TCC TTC AAC TAC GGT GCC ATC AAA GCG   540
LEU ALA GLN LYS VAL ALA ARG THR LEU PRO THR SER PHE ASN TYR GLY ALA ILE LYS ALA

ACC CGG GTC ACC GAG TTG CTT TAC CGG ATG AAG AGG GCC GAA ACA TAC TGT CCA AGG CCC   600
THR ARG VAL THR GLU LEU LEU TYR ARG MET LYS ARG ALA GLU THR TYR CYS PRO ARG PRO

TTG CTG GCA ATC CAC CCA ACT GAA GCC AGA CAC AAA CAG AAA ATT GTG GCA CCG GTG AAA   660
LEU LEU ALA ILE HIS PRO THR GLU ALA ARG HIS LYS GLN LYS ILE VAL ALA PRO VAL LYS

     ←VP1 | p52→
CAG ACT TTG|AAT TTT GAC CTT CTG AAG TTG GCA GGA GAC GTT GAG TCC AAC
GLN THR LEU|ASN PHE ASP LEU LEU LYS LEU ALA GLY ASP VAL GLU SER ASN
```

FIG. 2

```
                ←VP3|VP1→   10        20        30        40        50        60        70
(a) A12    LRLPIDPRSQTTATGESADPVTTTVENYGGETQVQRRHHTDVSFIMDRFVKIKSLNPTHVIDLMQTHQHGLVGALLRAAT
    A24                          TTTVENYGGETQIQRRHHTDIGFIMDRFVKIQSLSPTHVIDLMQTHQHGLVGALLRATT
    A10    LRLPIDPRTQTTITGESADPVTTTVENYGGDTQVQRRHHTDVGFIMDRFVKINSLSPTHVIDLMQTHKHGIVGALLRAAT

(b) A10    LRLPIDPRTQTTTTGESADPVTTTVENYGGDTQVQRRHHTDVGFIMDRFVKINSLSPTHVIDLMQTHKHGIVGALLRAAT
    C3     LRLPVDARLQTTTTGESADPVTTTVENYGGETQIQRRHHTDVAFVLDRFVKVHVSGNQHTLDVMQVHKDSIVGALLRAAT
    O1BFS  LRLPVDARAETTSAGESADPVTTTVENYGGETQIQRRQHTDVSFIMDRFVKVTPQNQINILDLMQVPSHTLVGALLRAST

(c) O1BFS  LRLPVDARAETTSAGESADPVTTTVENYGGETQIQRRQHTDVSFIMDRFVKVTPQNQINILDLMQVPSHTLVGALLRAST
    O1K    LRLPVDARAETTSAGESADPVTTTVENYGGETQIQRRQHTDVSFIMDRFVKVTPQNQINILDLMQIPSHTLVGALLRAST

                   80        90        100       110       120       130       140       150
(a) A12    YYFSDLEIVVRHDGNLTWVPNGAPEAALSNTGNPTAYNKAPFTRLALPYTAPHRVLATVYNGTNKYS--AS-GSGVRGDF
    A24    YYFSDLEIVVRHEGNLTWVPNGAPESALLNTSNPTAYNKAPFTRLALPYTAPHRVPATVYNGTSKYA--VG-GSGRRGDM
    A10    YYFSDLEIVVRHDGNLTWVPNGAPEAALSNTSNPTAYNKAPFTRLALPYTAPHRVLATVYDGTNKYS--AS-DS-RSGDL

(b) A10    YYFSDLEIVVRHDGNLTWVPNGAPEAALSNTSNPTAYNKAPFTRLALPYTAPHRVLATVYDGTNKYS--AS-DS-RSGDL
    C3     YYFSDLEIAVTHTGKLTWVPNGAPVSALDNTANPTAYHKGPLTRLALPYTAPHRVLATTYTGTTAYT--AS--A-RRGDL
    O1BFS  YYFSDLEIAVKHERDLTWVPNGAPEKALDNTTNPTAYHKAPLTRLALPYTAPHRVLATVYNGECRYSRNAVPNL-R-GDL

(c) O1BFS  YYFSDLEIAVKHERDLTWVPNGAPEKALDNTTNPTAYHKAPLTRLALPYTAPHRVLATVYNGECRYSRNAVPNL-R-GDL
    O1K    YYFSDLEIAVKHEGDLTWVPNGAPEKALDNTTNPTAYHKAPLTRLALPYTAPHRVLATVYNGECRYNRNAVPNL-R-GDL

                  160       170       180       190       200       210←VP1|p52→
(a) A12    GSLAFRVARQLPASFNYGAIKAETIHELLVRMKRAELYCPRPLLAIEVSSQDRHKQKIIAPGKQ-LNFDLLKLAGDVESN
    A24    GSLAARVVKQLPASFNYGAIKADAIHELLVRMKRAELYCPRPLLAIEVSSQDRHKQKIIAPAKQLLNFDLLKLAGDVESN
    A10    GSIAARVATQLPASFNYGAIQAQAIHELLVRMKRAELYCPRPLLAIKVTSQDRYKQKIIAPAKQLLNFDLLKLAGDVESN

(b) A10    GSIAARVATQLPASFNYGAIQAQAIHELLVRMKRAELYCPRPLLAIKVTSQDRYKQKIIAPAKQLLNFDLLKLAGDVESN
    C3     AHLAAAHARHLPTSFNFGAVKAETITELLVRMKRAELYCPRPVLPVQPT-GDRHKQPLIAPAKQLLNFDLLKLAGDVESN
    O1BFS  QVLAQKVARTLPTSFNYGAIKATRVTELLYRMKRAETYCPRPLLAIHPT-EARHKQKIVAPVKQTLNFDLLKLAGDVESN
                    †
(c) O1BFS  QVLAQKVARTLPTSFNYGAIKATRVTELLYRMKRAETYCPRPLLAIHPT-EARHKQKIVAPVKQTLNFDLLKLAGDVESN
    O1K    QVLAQKVARTLPTSFNYGAIKATRVTELLYRMKRAETYCPRPLLAIHPT-EARHKQKIVAPVKQTLNFDLLKLAGDVESN
```

FIG. 3

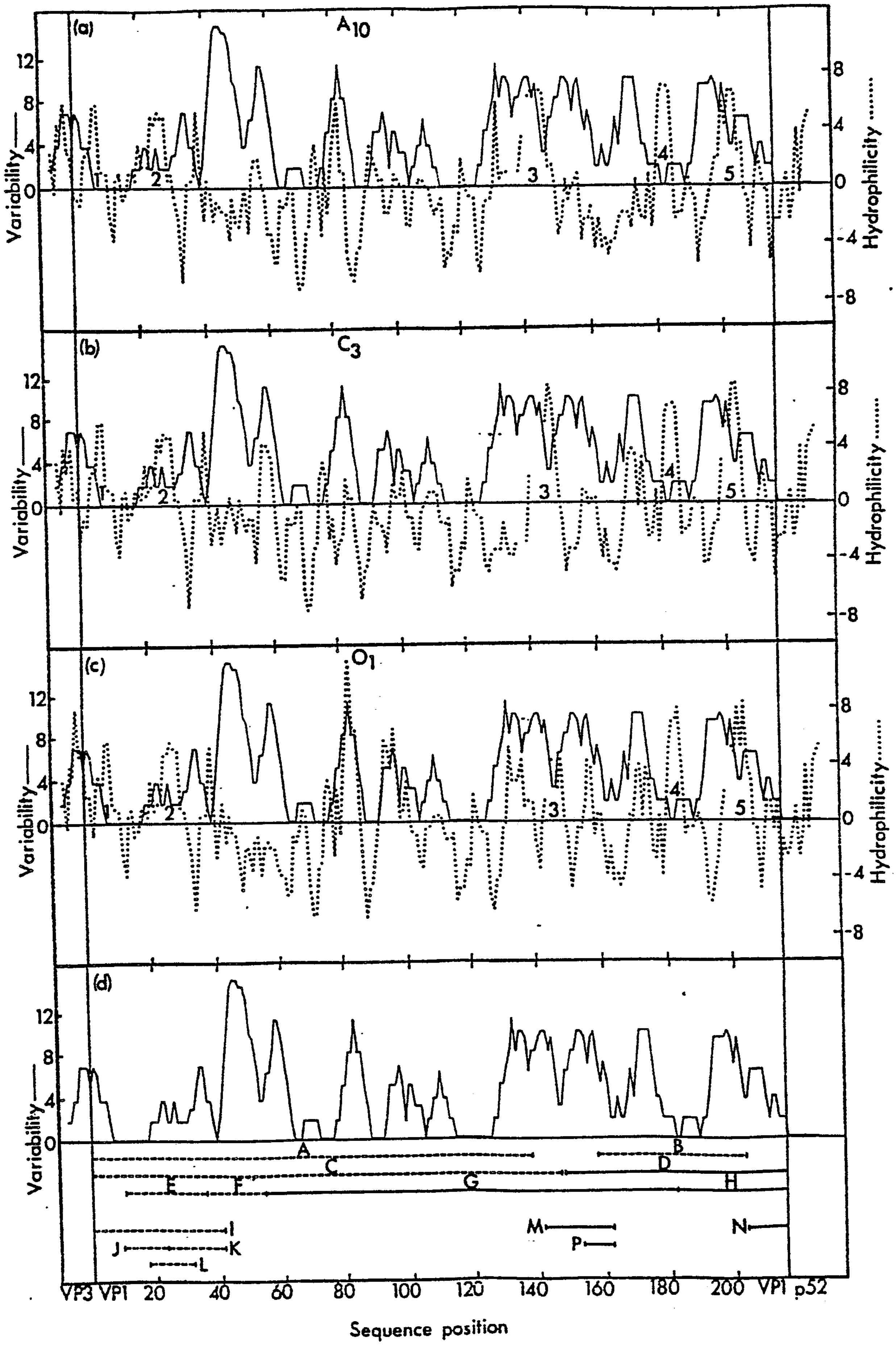
(a) A10
(b) C3
(c) O1
(d)
Variability
Hydrophilicity
A B C D E F G H I J K L M N P
VP3 VP1 20 40 60 80 100 120 140 160 180 200 VP1 p52
Sequence position

FIG. 4

0105481

European Patent Office

# EUROPEAN SEARCH REPORT

Application number

EP 83109769.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP - A1 - 0 044 710 (SCRIPPS CLINIC AND RESEARCH FOUNDATION) * Page 58, lines 15-18; claim 14; fig. 10 * | 1 | C 07 C 103/52<br>C 12 N 15/00<br>C 12 P 21/00<br>A 61 K 39/135<br>C 12 P 19/30 |
| X,P | EP - A2 - 0 068 693 (GENENTECH, INC.) * Abstract; claims 1-3,22; fig. 2, (part a) * | 1,10 | |
| X | EP - A1 - 0 040 922 (BIOGEN N.V.) * Abstract; claim 25; fig. 9 * | 1,12 | |
| X,D | NATURE, vol. 298, no. 5869, July 1, 1982, (New York, USA), J.L. BITTLE et al.: "Protection against foot-and-mouth disease by immunization with a chemically synthesized peptide predicted from the viral nucleotide sequence" pages 30-33 * Page 31; fig. 1 * | 1 | TECHNICAL FIELDS SEARCHED (Int. Cl. 3)<br>C 07 C 103/00<br>C 12 N<br>C 12 P<br>A 61 K |
| A,D | EP - A2 - 0 048 455 (THE WELLCOME FOUNDATIN LIMITED) * Claims 1,5,26,28 * | 12,16, 17 | |
| A | US - A - 4 140 763 (BACHRACH et al.) * Abstract; claims 1-3 * | 8,10, 18 | |

The present search report has been drawn up for all claims

| Place of search | Date of completion of the search | Examiner |
|---|---|---|
| VIENNA | 30-12-1983 | WOLF |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82